(19) Europäisches Patentamt
European Patent Office
Office européen des brevets

(11) Veröffentlichungsnummer: **0 171 724**
**A2**

# (12) EUROPÄISCHE PATENTANMELDUNG

(21) Anmeldenummer: 85109777.4

(22) Anmeldetag: 03.08.85

(51) Int. Cl.⁴: C 07 D 263/58
C 07 D 277/68, C 07 D 215/20
C 07 D 241/44, A 01 N 43/76
A 01 N 43/78, A 01 N 43/42
A 01 N 43/60

(30) Priorität: 17.08.84 DE 3430215

(43) Veröffentlichungstag der Anmeldung:
19.02.86 Patentblatt 86/8

(84) Benannte Vertragsstaaten:
AT BE CH DE FR GB IT LI NL

(71) Anmelder: **HOECHST AKTIENGESELLSCHAFT**
**Postfach 80 03 20**
**D-6230 Frankfurt am Main 80(DE)**

(72) Erfinder: **Handte, Reinhard, Dr.**
**Theilweg 23**
**D-8901 Gablingen(DE)**

(72) Erfinder: **Mildenberger, Hilmar, Dr.**
**Fasanenstrasse 24**
**D-6233 Kelkheim (Taunus)(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(54) **Phenoxypropionsäurederivate, Verfahren zu ihrer Herstellung und ihre Verwendung als Herbizide.**

(57) Die Verbindungen der Formel I

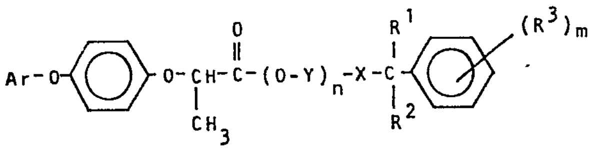 (I)

worin

Ar die Gruppe

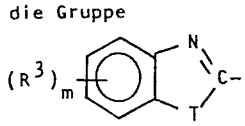 oder 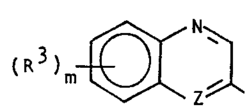 ,

$R^1$ H oder Alkyl, $R^2$ H, Alkyl oder Phenyl, $R^3$ H, Halogen, Nitro, Alkyl, Alkoxy oder $CF_3$; X, T O, S oder $N-R^1$, m 1 oder 2, n 1, 2, 3 oder 4, Y eine Alkylen-, Alkenylen- oder Alkinylenkette und Z CH oder N bedeuten, besitzen vorteilhafte selektive Herbizidwirkungen gegen ein- und mehrjährige Schadgräser. Außerdem weisen die Verbindungen der Formel I pflanzenwachstumsregulierende Wirkungen auf.

EP 0 171 724 A2

Phenoxypropionsäurederivate, Verfahren zu ihrer Herstellung und ihre Verwendung als Herbizide

Gegenstand der vorliegenden Erfindung sind substituierte Phenoxypropionsäurederivate der Formel I

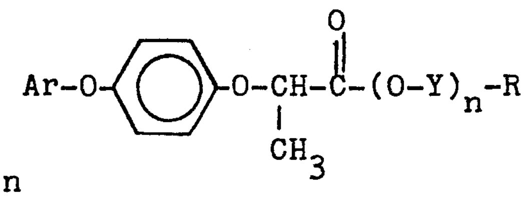

(I)

worin

Ar die Gruppe

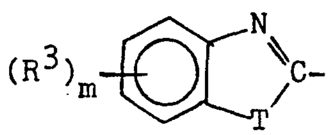

oder

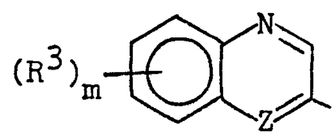

,

Y eine verzweigte oder geradkettige $(C_2-C_{12})$-Alkylen-, $(C_3-C_{12})$-Alkenylen- oder $(C_3-C_{12})$-Alkinylenkette,

R einen Rest der Formel

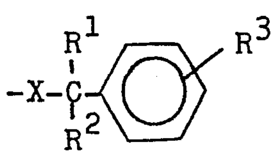

oder $-A-Si[(C_1-C_4\text{-alkyl})]_3$,

$R^1$ Wasserstoff oder $(C_1-C_6)$-Alkyl,

$R^2$ Wasserstoff, $(C_1-C_6)$-Alkyl oder Phenyl,

$R^3$ unabhängig voneinander Wasserstoff, Halogen, Nitro, $(C_1-C_6)$-Alkyl, $(C_1-C_6)$-Alkoxy oder $CF_3$,

X, T unabhängig voneinander O, S oder $N-R^1$,

A O, S, $NR^1$ oder eine direkte Bindung,

m 1 oder 2

n 1, 2, 3 oder 4,

Z CH oder N bedeuten.

Bevorzugte Verbindungen der Formel I sind diejenigen, in denen

Ar die Gruppe

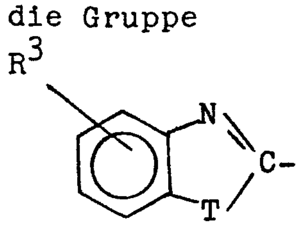 oder 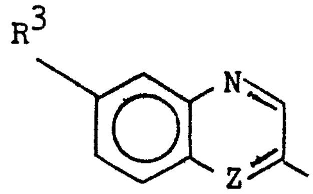 ,

R den Rest 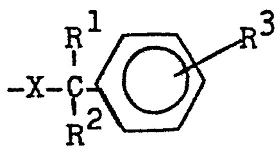

$R^1$ Wasserstoff oder $(C_1-C_6)$-Alkyl

$R^2$ Wasserstoff

$R^3$ Wasserstoff, Fluor, Chlor, Brom (oder $CF_3$) K.B.

T O oder S,

X O

Z CH oder N,

n 1, 2, 3 oder 4

Y eine geradkettige oder verzweigte $(C_2-C_{12})$-Alkylenkette

bedeuten.

Insbesondere werden dabei solche Verbindungen bevorzugt, in denen Y für die $-\underset{R^4}{CH}-(CH_2)_p-$ Gruppierung steht, in der

$R^4$ Wasserstoff oder Methyl und

p 1 oder 2 bedeuten, und n in Formel I für die Zahl 1 und $R_2$ für H steht.

Die Verbindungen der allgemeinen Formel I besitzen ein Asymmetriezentrum und werden bei der Herstellung gewöhnlich als Racemate erhalten. Die Erfindung umfaßt jedoch auch die isolierten optischen Antipoden und dabei insbesondere deren D-Formen.

Gegenstand der vorliegenden Erfindung ist weiter ein Verfahren zur Herstellung der Verbindungen der Formel I, dadurch gekennzeichnet, daß man

a) eine Verbindung der Formel II

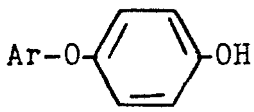

(II)

mit einer Verbindung der Formel III,

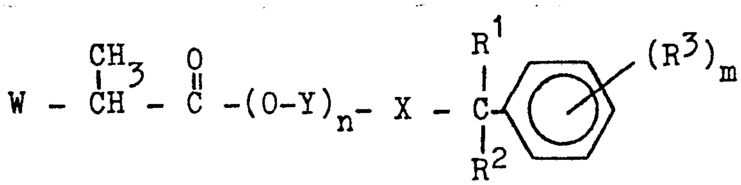

(III)

worin W für ein Halogenatom, eine Mesyl- oder Tosylgruppe steht,

b) eine Verbindung der Formel IV, worin

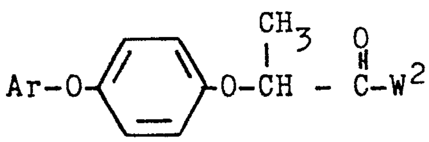

(IV)

$W^2$ Hydroxy oder Halogen bedeutet,

mit einer Verbindung der Formel V

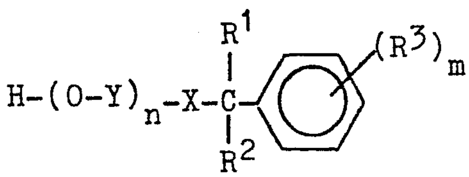

(V)

oder

c) eine Verbindung der Formel VI

Ar-W (VI)

mit einer Verbindung der Formel VII

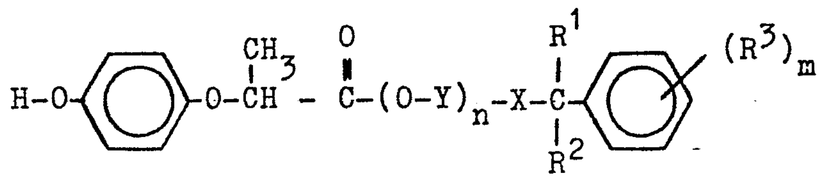

(VII)

umsetzt.

Die Reaktionsvariante a) gemäß der vorliegenden Erfindung wird vorzugsweise in Gegenwart eines Verdünnungsmittels durchgeführt. Zu diesem Zweck werden inerte Lösungsmittel verwendet.

Beispiele für solche Lösungsmittel oder Verdünnungsmittel sind aliphatische, alicyclische oder bevorzugt aromatische Kohlenwasserstoffe, die gegebenenfalls chloriert sein können, wie Toluol, Xylol, Methylenchlorid, Kohlenstofftetrachlorid, Ethylenchlorid, Trichloroethylen, Chlorbenzol und Dichlorbenzol, ferner Ether - Verbindungen wie Diethylether oder Tetrahydrofuran, Ketone wie Aceton, Methylethylisopropylketon, Methylisobutylketon, Nitrile wie Acetonitril, Säureamide wie Dimethylformamid, N-Methylpyrrolidon und Dimethylacetamid, Sulfone oder Sulfoxide wie Dimethylsulfoxid oder Sulfolan.

Die Reaktion wird vorzugsweise in Gegenwart eines säurebindenden Mittels bei Temperaturen zwischen 20°C und 160°C oder dem Siedepunkt der Reaktionsmischung durchgeführt. Üblicherweise wird zur Umsetzung bei Normaldruck gearbeitet, es ist jedoch auch möglich, erhöhten oder verminderten Druck anzuwenden. Als Beispiele für solche säurebindenden Mittel seien Hydroxide, bevorzugt Alkoholate, Carbonate und Hydrogencarbonate von Alkalimetallen insbesondere von Natrium und Kalium, genannt. Zur Erhöhung der Reaktivität ist es vorteilhaft, falls W für Chlor steht,

in Anwesenheit von Natrium- oder Kaliumjodid zu arbeiten, in der Regel sind dabei Mengen zwischen 1 und 10 Mol-% bezogen auf die Komponente II ausreichend.

Bei der Durchführung der Reaktionsvariante b) wird eines der im vorstehenden für die Variante a) beschriebenen Lösungsmittel oder Verdünnungsmittel verwendet. Es wird gleichermaßen vorzugsweise in Gegenwart eines säurebindenden Mittels, wie sie für die Variante a) beschrieben sind, gearbeitet. Hierbei eignen sich auch tert. Amine als Basen. Bezüglich Druck und Temperatur entsprechen die Reaktionsbedingungen denen der Umsetzung nach a). Für die bevorzugte Reaktionsvariante c) gelten ebenfalls die unter a) genannten Reaktionsparameter. Es hat sich dabei gezeigt, daß es vorteilhaft ist, die Umsetzung in Gegenwart eines Katalysators aus der Gruppe der quartären Ammonium- oder Phosphoniumbasen, der Polyalkylenglykole oder deren Niederalkylether durchzuführen.

Die Katalysatoren werden in Mengen von 0,05 bis 1,0 Mol-%, bevorzugt 0,1 bis 0,5 Mol-%, bezogen auf die Komponente IV oder V, angewendet. Die quartären Ammonium- und Phosphoniumbasen enthalten als organische Reste Alkyl, Benzyl oder Phenylgruppen und liegen üblicherweise als Halogenide vor. Als Beispiele seine genannt: Tetrabutylammoniumbromid, Tetrabutylammoniumjodid, Triethylbenzylammoniumchlorid, Tetraoctylphosphoniumchlorid, Tetrabutylphosphoniumbromid, Triphenylbutylphosphoniumbromid, Trilaurylmethylphosphoniumjodid, Tributylmethylphosphoniumjodid und Tributylhexadecylphosphoniumbromid.

Daneben eignen sich cyclische Polyethylenglykole (Kronenether) und langkettige Polypropylen- und besonders Polyethylenglykole mit Molekulargewichten von ca. 400 - ca. 6000 sowie deren Mono- und Diniederalkylether. Verbindungen dieser Art sind in der organischen Chemie allgemein unter der Bezeichnung "Phasentransferkatalysatoren" bekannt, da sie Reaktionen im Zweiphasensystem Wasser/organisches Lösungsmittel beschleunigen.

Als säurebindende Mittel werden bevorzugt Alkalicarbonate und -hydrogencarbonate, insbesondere die Kalium- und Natriumsalze verwendet.

Die Ausgangsverbindungen der Formeln II - VII sind bekannt bzw. lassen sich nach bekannten Verfahren herstellen. Bei Verwendung von optisch aktiven Ausgangsmaterialien der Formeln III, IV oder VII ist es möglich, optische Isomere der erfindungsgemäßen Verbindungen (bevorzugt die D-Form) der allgemeinen Formel I in hohen optischen Reinheiten herzustellen.

Die erfindungsgemäßen Verbindungen der allgemeinen Formel I sind im Vor- und insbesondere im Nachauflaufverfahren gegen ein breites Spektrum von ein- und mehrjährigen Schadgräsern sehr gut wirksam; gleichzeitig werden sie jedoch von zweikeimblättrigen Kulturpflanzen sowie teilweise von einigen Getreidearten (wie z. B.Weizen, Gersteu.a.) vorzüglich toleriert. Die Verbindungen sind daher zur selektiven Bekämpfung von ein- und mehrjährigen Schadgräsern in Kulturpflanzen geeignet. Solche Schadgräser sind beispielsweise Wildhafer (Avena), Fuchsschwanz (Alopecurus spp.), Rispengras (Poa spp.), Raygras (Lolium spp.), ein- und mehrjährige Wildhirsen (Echinochloa spp., Setaria spp., Digitaria spp., Panicum spp., Sorghum spp.), Bermudagras (Cynodon spp.) und Quecke (Agropyron spp.).

...

In subletalen Dosen zeichnen sich die erfindungsgemäßen Verbindungen durch wachstumsregulierende Eigenschaften aus. Es lassen sich z. B. der Wuchs oder die Inhaltsstoffe von Pflanzen (wie Hirsen, Zuckerrohr, Zuckerrüben u. a.) durch Anwendung der Verbindungen gezielt beeinflussen.

Gegenstand der vorliegenden Erfindung sind daher auch herbizide und wachstumsregulierende Mittel, die dadurch gekennzeichnet sind, daß sie eine herbizid wirksame Menge einer Verbindung der allgemeinen Formel I neben üblichen Zusatz- und Formulierungshilfsmitteln enthalten.

Die erfindungsgemäßen Mittel enthalten die Wirkstoffe der Formel I im allgemeinen zu 2 - 95 Gew.-%. Sie können als benetzbare Pulver, emulgierbare Konzentrate, versprühbare Lösungen, Stäubemittel oder Granulate in den üblichen Zubereitungen angewendet werden.

Benetzbare Pulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Oleyl-, Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, oder auch oleylmethyltaurinsaures Natrium enthalten.

Emulgierbare Konzentrate werden durch Auflösen des Wirkstoffes in einem organischen Lösungsmittel, z. B. Butanol, Cylcohexanon, Dimethylformamid, Xylol oder auch höhersiedenden Aromaten und Zusatz eines nichtionischen Netzmittels, beispielsweise eines polyoxethylierten Alkylphenols oder eines polyoxethylierten Oleyl- oder Stearylamins, erhalten.

...

Stäubemittel erhält man durch Vermahlen des Wirkstoffes mit feinverteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium, oder auch Mineralölen auf die Oberfläche von Trägerstoffen, wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei den herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10 % und 95 %, der Rest besteht aus den oben angegebenen Formulierungszusätzen. Bei emulgierbaren Konzentraten ist die Wirkstoffkonzentration etwa 10 % bis 80 %. Staubförmige Formulierungen enthalten meistens 5 % bis 20 % an Wirkstoff. Bei Granulaten hängt der Wirkstoffgehalt z. T. davon ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z. B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u. a. variiert die erforderliche Aufwandmenge. Sie kann innerhalb weiter Grenzen schwanken, z. B. zwischen 0,01 und 10,0 kg/ha

...

oder mehr Aktivsubstanz, vorzugsweise liegt sie jedoch zwischen 0,05 und 5 kg/ha.

Die erfindungsgemäßen Wirkstoffe können mit anderen Wachstumsregulatoren, Herbiziden, Insektiziden und Fungiziden kombiniert werden.

Bei der Kombination mit Herbiziden eignen sich insbesondere die gegen Dikotyledonen wirksamen Präparate aus der Gruppe der Nitrodiphenylether, der Sulfonylharnstoffe sowie Phenmedipham, Desmedipham, Bentazon und Metamitron.

Formulierungsbeispiele

Beispiel A

Ein emulgierbares Konzentrat wird erhalten aus
15 Gew.-Teilen Wirkstoff
75 Gew.-Teilen Cyclohexanon als Lösungsmittel
und 10 Gew.-Teilen oxethyliertes Nonylphenol (10 AeO) als Emulgator.

Beispiel B

Ein in Wasser leicht dispergierbares benetzbares Pulver wird erhalten, indem man
25 Gew.-Teile Wirkstoff
64 Gew.-Teile kaolinhaltiges Quarz als Inertstoff
10 Gew.-Teile ligninsulfonsaures Kalium
und 1 Gew.-Teil oleylmethyltaurinsaures Natrium als Netz- und Dispergiermittel
mischt und in einer Stiftmühle mahlt.

...

Beispiel C

Ein Stäubemittel wird erhalten, indem man
10 Gew.-Teile Wirkstoff
und 90 Gew.-Teile Talkum als Inertstoff
mischt und in einer Schlagmühle zerkleinert.

Beispiel D

Ein Granulat besteht z. B. aus etwa
2 - 15 Gew.-Teilen Wirkstoff
98 - 85 Gew.-Teilen inerten Granulatmaterialien, wie z. B. Attapulgit, Bimsstein und Quarzsand.

....

## Chemische Beispiele

### Beispiel 1

2-Benzyloxyethyl-2-[4-(6-chlor-2-benzoxazolyloxy)-phenoxy]-propionat

33,4 g 2-[4-(6-Chlor-2-benzoxazolyloxy)-phenoxy]- propionsäure werden in 250 ml trockenem Toluol vorgelegt, dazu tropft man 13,1 g Thionylchlorid. Bis zum Ende der Gasentwicklung wird bei 70 °C nachgerührt. Nach Abdestillieren des überschüssigen Thionylchlorids werden bei Raumtemperatur 11,6 g Triethylamin und 16 g Benzyloxyethanol zugefügt. Nach der Zugabe wird 2 h bei 40 °C nachgerührt, auf Raumtemperatur abgekühlt, zweimal mit je 150 ml 5%iger Natronlauge und einmal mit 150 ml Wasser gewaschen. Nach Trocknen über Magnesiumsulfat und Abdestillieren des Toluols, verbleiben 43 g (92 % der Theorie) an 2-Benzyloxyethyl-2-[4-(6-chlor-2-benzoxyazolyloxy)-phenoxy]-propionat in Form eines zähen Öles.

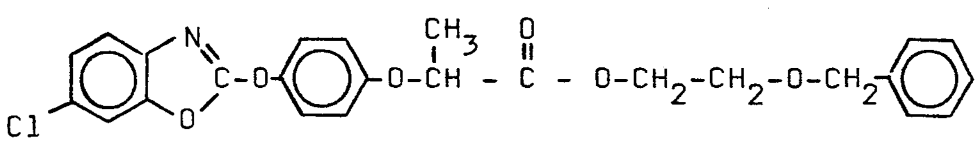

### Beispiel 2

2-(2-Chlorbenzyloxyethyl)-2-[4-(6-chlor-2-benzoxazolyloxy)-phenoxy]-propionat

19 g 2,6-Dichlorbenzoxazol, 39,2 g 2-(2-Chlorbenzyloxyethyl-2-(4-hydroxyphenoxy)-propionat, 13,8 g Kaliumcarbonat und 1 g Polyethylenglykol 2000 werden unter leicht reduziertem Druck 3 h bei 120 °C gerührt. Nach Aufarbeitung, wie in Beispiel 1 beschrieben, erhält man

...

47,4 g (94,4 % der Theorie) an 2-(2-Chlorbenzyloxyethyl)-2-[4-(6-chlor-2-benzoxazolyloxy)-phenoxy]-propionat als zähes Öl.

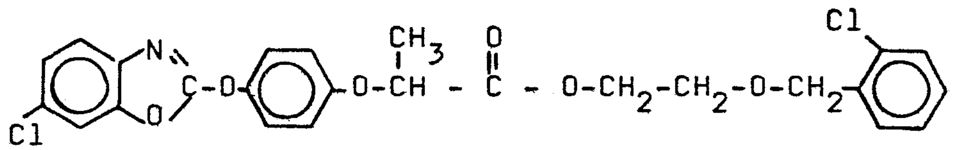

Beispiel 3

2-(2-Chlorbenzyloxyethyl)-2-[4-(5-chlor-2-benzoxazolyl-oxy)-phenoxy]-propionat

19 g 2,5-Dichlorbenzoxazol, 39,2 g 2-(2-Chlorbenzyloxy-ethyl)-2-(4-hydroxyphenoxy)-propionat und 16,6 g Kaliumcarbonat werden in 250 ml Acetonitril 8 h zum Sieden erhitzt. Nach Abkühlen wird abgesaugt, Acetonitril abdestilliert und der Rückstand in 350 ml Toluol gelöst. Anschließend wird zweimal mit je 150 ml 5%iger Natronlauge und einmal mit 150 ml 5%iger Natriumdihydrogenphosphatlösung gewaschen. Nach Einengen verbleiben 45,7 g (91 % der Theorie) an 2-(2-Chlorbenzyloxyethyl)-2-[4-(5-chlor-2-benzoxazolyl-oxy)-phenoxy]-propionat als Öl.

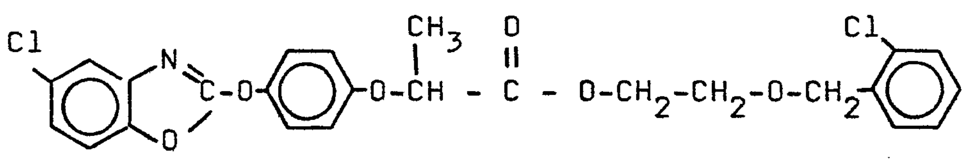

...

0171724

Beispiel 4

2-(4-Chlorbenzyloxyethyl)-2-[4-(6-chlor-2-benzthiazolyl-oxy)-phenoxy]-propionat

27,8 g 4-(6-Chlor-2-benzothiazolyloxy)-phenol, 41,5 g 2-(4-Chlorbenzyloxyethyl)-2-chlorpropionat, 27,6 g Kaliumcarbonat und 3 g Natriumjodid werden in 300 ml N-Methylpyrrolidon ca. 15 min bei 140 °C gerührt. Nach Abkühlen wird abgesaugt und anschließend im Vakuum eingeengt. Der verbleibende Rückstand wird in 400 ml Toluol aufgenommen und wie zuvor beschrieben aufgearbeitet. Man erhält 44 g (84,9 % der Theorie) an 2-(4-Chlorbenzyloxyethyl)-2-[4-(6-chlor-2-benzothiazolyloxy)-phenoxy]-propionat, Fp. 67 - 69°.

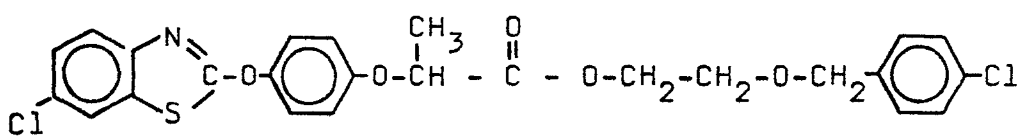

Beispiel 5

2-(4-Fluorbenzyloxyethyl)-2-[4-(6-chlor-2-chinoxalyloxy)-phenoxy]-propionat

20 g 2,6-Dichlorchinoxalin, 36,7 g 2-(4-Fluorbenzyloxyethyl)-2-(4-hydroxyphenoxy)-propionat und 16,6 g Kaliumcarbonat werden 24 h in 350 ml Acetonitril zum Sieden erhitzt. Nach Abkühlen werden die Salzanteile abgesaugt, Acetonitril abdestilliert und der Rückstand in 300 ml Toluol aufgenommen. Nach Aufarbeitung wie in Beispiel 3, verbleiben als Rückstand 41,4 g (86 % der Theorie) an 2-(4-Fluorbenzyloxyethyl)-2-[4-(6-chlor-2-chinoxalyloxy)-phenoxy]-propionat als Öl.

...

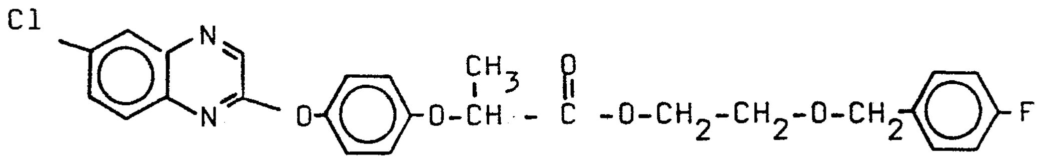

In analoger Weise werden die in nachfolgender Tabelle angegebenen Verbindungen hergestellt.

Tabelle

Verbindungen der allgemeinen Formel

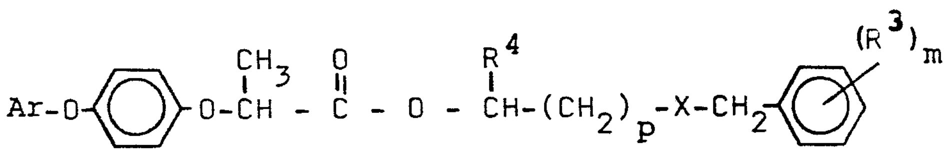

| Bsp. Nr. | Ar | $R^4$ | p | X | $(R^3)_m$ |
|---|---|---|---|---|---|
| 6 | 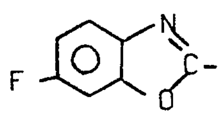 | H | 1 | O | H |
| 7 | " | H | 1 | O | 4-Cl |
| 8 | " | H | 1 | O | 3-Cl |
| 9 | " | H | 1 | O | 2-Cl |
| 10 | " | H | 1 | O | 2,6-Di-Cl |
| 11 | 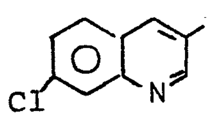 | H | 1 | O | H |
| 12 | " | H | 1 | O | 4-Cl |
| 13 | 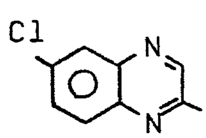 | H | 1 | O | H |

Fortsetzung Tabelle

| Bsp. Nr. | Ar | $R^4$ | p | X | $(R^3)_m$ | Fp.(°C) |
|---|---|---|---|---|---|---|
| 14 | 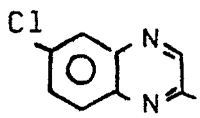 | H | 1 | O | 4-Cl | |
| 15 | " | H | 1 | O | 2-$CH_3$ | |
| 16 | " | H | 1 | O | 3-Cl | |
| 17 | " | H | 1 | O | 2,4-Di-Cl | |
| 18 | " | $CH_3$ | 1 | O | H | |
| 19 | " | " | 1 | O | 2-F | |
| 20 | " | H | 2 | O | H | |
| 21 | 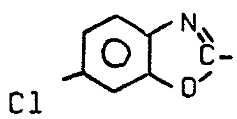 | H | 1 | O | 3-Cl | 71 - 75 |
| 22 | " | H | 1 | O | 4-Cl | 60 - 62 |
| 23 | " | H | 1 | O | 2-F | |
| 24 | " | H | 1 | O | 3-$OCH_3$ | Oel |
| 25 | " | H | 1 | O | 2,4-Di-Cl | 70 - 75 |
| 26 | " | H | 1 | O | 2,6-Di-Cl | 77 - 81 |
| 27 | " | H | 1 | O | 2-$CH_3$ | Oel |
| 28 | " | H | 1 | O | $CF_3$ | Oel |
| 29 | " | H | 1 | O | 3,5-Di-Cl | 75 - 79 |
| 30 | " | $CH_3$ | 1 | O | 3-Cl | |
| 31 | " | H | 1 | S | H | 79 - 83 |
| 32 | " | H | 1 | S | 4-Cl | |
| 33 | " | H | 2 | O | H | |
| 34 | " | H | 2 | O | 2-Cl | |
| 35 | 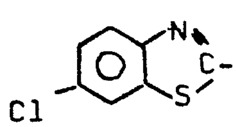 | H | 1 | O | H | 60 - 64 |
| 36 | " | H | 1 | O | 2-Cl | Oel |

Forsetzung Tabelle

| Bsp. Nr. | Ar | $R^4$ | p | X | $(R^3)_m$ | Fp.(°C) |
|---|---|---|---|---|---|---|
| 37 | 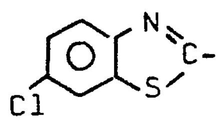 | H | 1 | O | 3-Cl | Oel |
| 38 | " | H | 1 | O | 2,4-Di-Cl | 69-71 |
| 39 | " | H | 1 | O | $2-CH_3$ | |
| 40 | " | H | 1 | O | $3-OCH_3$ | Oel |
| 41 | " | H | 1 | O | 4-F | |
| 42 | " | $CH_3$ | 1 | O | H | |
| 43 | " | " | 1 | O | 2-Cl | |
| 44 | " | H | 1 | O | 3,5-DiCl | Oel |
| 45 | " | $CH_3$ | 1 | O | 4-Cl | |
| 46 | " | H | 1 | S | H | Oel |
| 47 | " | H | 1 | S | 4-Cl | |
| 48 | 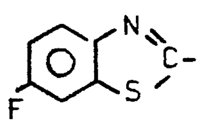 | H | 1 | O | H | |
| 49 | " | H | 1 | O | 3-Cl | |
| 50 | 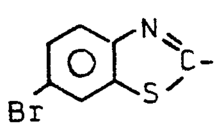 | H | 1 | O | H | |
| 51 | 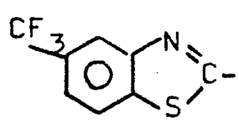 | H | 1 | O | H | |
| 52 | 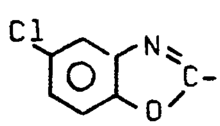 | H | 1 | O | H | |
| 53 | " | H | 1 | O | 4-Cl | |

Beispiel 54

In einer Lösung von 12,3 g (0,035 mol) 2-[4-(6-Chlor-2-benzoxazolyloxy)-phenoxy]-propionsäurechlorid und 4,2 g (0,042 Mol) Triethylamin in 100 mol trockenem Toluol gibt man bei 25°C 5 g (0,038 mol) 3-Trimethylsilyl-1-propanol hinzu. Nach der Zugabe wird 3 h bei 60°C nachgerührt, die Lösung vom Salz abgesaugt, je zweimal mit 100 ml 5 %iger Natronlauge und 100 ml Wasser gewaschen, getrocknet und zur Trockene eingeengt. Man erhält 14,2 g (90,5 % d. Theorie) an 2-(3-Trimethylsilylprop-1-yl)-[4-(6-Chlor-2-benzoxazolyloxy)-phenoxy]-propionat als Öl mit einem Brechungsindex $n_D^{24}$= 1,5366.

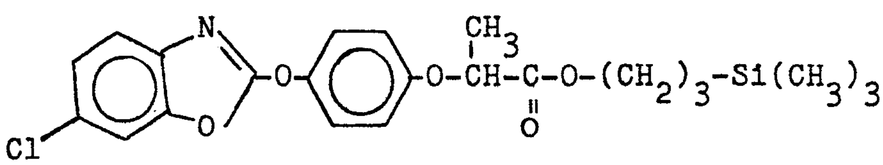

## Biologische Beispiele

### Beispiel I

Vorauflaufbehandlung

Samen von Gräsern (Flughafer, Ackerfuchsschwanz, Borstenhirse, Raygras, Hühnerhirse, Quecke und Bermudagras) wurden in Töpfen ausgesät und die als Spritzpulver bzw. als Emulsionskonzentrate formulierten erfindungsgemäßen Präparate in verschiedenen Dosierungen (2,4 kg a. i./ha und 0,6 kg a. i./ha) auf die Erdoberfläche gesprüht. Anschließend wurden die Töpfe für 4 Wochen in einem Gewächshaus aufgestellt und das Resultat der Behandlung (ebenso wie bei den folgenden Beispielen) durch eine Bonitierung festgehalten.

Die erfindungsgemäßen Präparate zeigten eine gute Wirkung gegen einjährige und zum Teil auch mehrjährige Schadgräser.

### Beispiel II

Nachauflaufbehandlung

Samen von Gräsern (Flughafer, Ackerfuchsschwanz, Borstenhirse, Raygras, Hühnerhirse, Quecke und Bermudagras) wurden in Töpfen ausgesät und im Gewächshaus angezogen. 3 Wochen nach der Aussaat wurden die als Spritzpulver bzw. als Emulsionskonzentrate formulierten erfindungsgemäßen Präparate in verschiedenen Dosierungen (2,4 und 0,6 kg a.i./ha) auf die Pflanzen gesprüht und nach 4 Wochen Standzeit im Gewächshaus die Wirkung der Präparate bonitiert.

Die erfindungsgemäßen Mittel waren gut gegen ein breites Spektrum von einjährigen Schadgräsern herbizid wirksam.

...

Einige Präparate bekämpften ferner auch die mehrjährigen Schadgräser Cynodon dactylon, Sorghum halepense sowie Agropyron repens.

Beispiel III

Kulturpflanzenverträglichkeit

In weiteren Versuchen im Gewächshaus wurden Samen einer größeren Anzahl von Kulturpflanzen in Töpfen ausgelegt. Ein Teil der Töpfe wurde sofort behandelt; die übrigen wurden im Gewächshaus aufgestellt, bis die Pflanzen 2 bis 3 echte Blätter entwickelt hatten und dann mit den erfindungsgemäßen Substanzen besprüht.
Nach 4 bis 5 Wochen nach Applikation wurde festgestellt, daß die erfindungsgemäßen Substanzen zweikeimblättrige Kulturen im Vor- und Nachauflaufverfahren bei Dosierungen bis zu 2,5 kg/ha Wirkstoff ungeschädigt ließen. Einige Substanzen schonten darüber hinaus auch Gramineen-Kulturen wie Gerste, Sorghum, Mais, Weizen oder Reis. Die Verbindungen der Formel I weisen somit eine hohe Selektivität bei ihrer Anwendung auf.

Bonitierung in %-Werten und Zahlen

| Wertzahl | Schadwirkung in % bei Unkräutern und Kulturpflanzen |
|---|---|
| 1 | 0 - 20 % |
| 2 | 20 - 40 % |
| 3 | 40 - 60 % |
| 4 | 60 - 80 % |
| 5 | 80 - 100 % |

Patentansprüche:

1. Verbindungen der allgemeinen Formel I

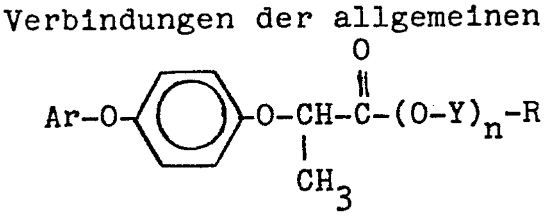

(I)

worin

Ar die Gruppe

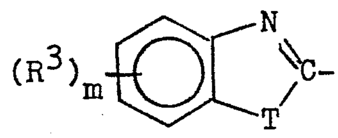

oder

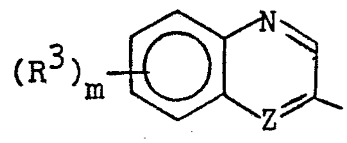

,

Y eine verzweigte oder geradkettige $(C_2-C_{12})$-Alkylen-, $(C_3-C_{12})$-Alkenylen- oder $(C_3-C_{12})$-Alkinylenkette

R einen Rest der Formel

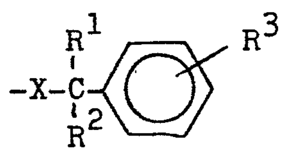

oder $-A-Si[(C_1-C_4)alkyl]_3$,

$R^1$ Wasserstoff oder $(C_1-C_6)$-Alkyl,

$R^2$ Wasserstoff, $(C_1-C_6)$-Alkyl oder Phenyl,

$R^3$ unabhängig voneinander Wasserstoff, Halogen, Nitro, $(C_1-C_6)$-Alkyl, $(C_1-C_6)$-Alkoxy oder $CF_3$,

X, T unabhängig voneinander O, S oder $N-R^{13}$,

A O, S, $N-R^1$ oder eine direkte Bindung

m 1 oder 2

n 1, 2, 3 oder 4,

Y eine verzweigte oder geradkettige $(C_2-C_{12})$-Alkylen-, $(C_3-C_{12})$-Alkenylen- oder $(C_3-C_{12})$-Alkinylenkette und

Z CH oder N bedeuten.

2. Verbindungen gemäß Anspruch 1, dadurch gekennzeichnet, daß

Ar die Gruppe

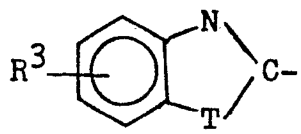

oder

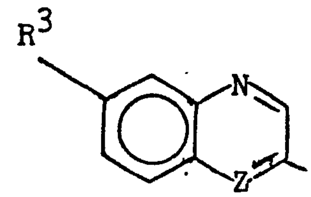

R den Rest 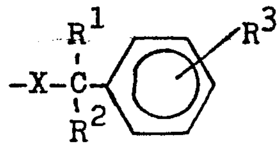

$R^1$ Wasserstoff oder $(C_1-C_6)$-Alkyl
$R^2$ Wasserstoff
$R^3$ Wasserstoff, Fluor, Chlor, Brom oder $CF_3$,
T O oder S,
X O
Z CH oder N,
n 1, 2, 3 oder 4 und
Y eine geradkettige oder verzweigte $(C_2-C_{12})$-Alkylenkette

bedeuten.

3. Verbindungen gemäß Anspruch 2, dadurch gekennzeichnet, daß n 1 und $R_2$ Wasserstoff sind und Y für eine Gruppierung $-\underset{R^4}{CH}-(CH_2)_p-$ steht, in der

$R^4$ Wasserstoff oder Methyl und
p 1 oder 2 bedeuten.

4. Verfahren zur Herstellung der Verbindungen von Anspruch 1, dadurch gekennzeichnet, daß man

a) eine Verbindung der Formel II

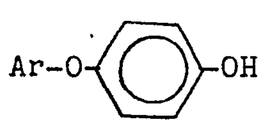

(II)

mit einer Verbindung der Formel III,

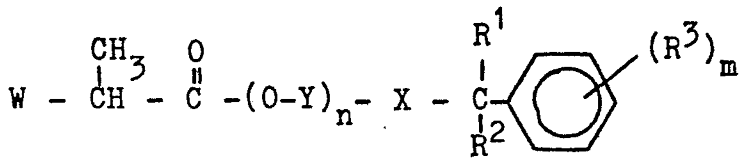 (III)

worin W für ein Halogenatom, eine Mesyl- oder Tosylgruppe steht, oder

b) eine Verbindung der Formel IV

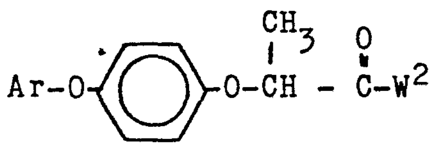 (IV)

worin $W^2$ für eine Hydroxylgruppe oder Halogenatom steht, mit einer Verbindung der Formel V

mit einer Verbindung der Formel V

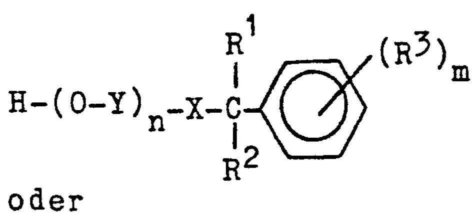 (V)

oder

c) eine Verbindung der Formel VI

Ar-W (VI)

mit einer Verbindung der Formel VII

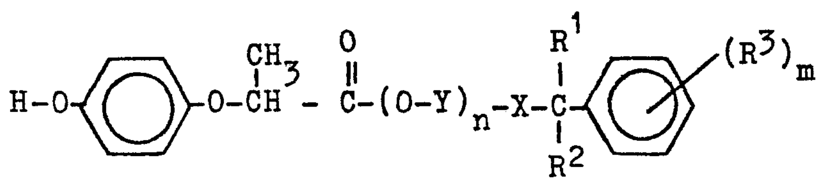 (VII)

umsetzt.

5. Herbizide Mittel, die gekennzeichnet sind durch einen wirksamen Gehalt an einer Verbindung gemäß Ansprüchen 1 bis 3.

6. Verfahren zur Bekämpfung von unerwünschtem Pflanzenwuchs bzw. zur Wachstumsregulierung von Nutzpflanzen, dadurch gekennzeichnet, daß die Pflanzen oder die Anbauflächen mit einer wirksamen Menge einer Verbindung der Formel I behandelt werden.

7. Wachstumsregulierende Mittel, die gekennzeichnet sind durch einen wirksamen Gehalt an einer Verbindung gemäß Ansprüchen 1 bis 3.

Ansprüche Österreich

1. Herbizide und wachstumsregulierende Mittel, gekennzeichnet durch einen wirksamen Gehalt an einer Verbindung der Formel I

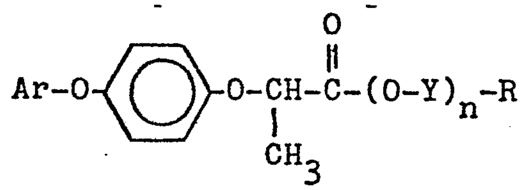

(I)

worin

Ar die Gruppe

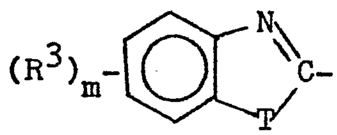

oder

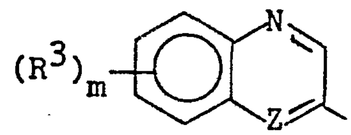

,

Y eine verzweigte oder geradkettige $(C_2-C_{12})$-Alkylen-, $(C_3-C_{12})$-Alkenylen- oder $(C_3-C_{12})$-Alkinylenkette

R einen Rest der Formel

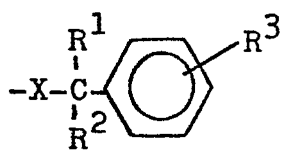

oder $-A-Si[(C_1-C_4)alkyl]_3$,

$R^1$ Wasserstoff oder $(C_1-C_6)$-Alkyl,

$R^2$ Wasserstoff, $(C_1-C_6)$-Alkyl oder Phenyl,

$R^3$ unabhängig voneinander Wasserstoff, Halogen, Nitro, $(C_1-C_6)$-Alkyl, $(C_1-C_6)$-Alkoxy oder $CF_3$,

X, T unabhängig voneinander O, S oder $N-R^1$,

A O, S, $N-R^1$ oder eine direkte Bindung

m 1 oder 2

n 1, 2, 3 oder 4,

Y eine verzweigte oder geradkettige $(C_2-C_{12})$-Alkylen-, $(C_3-C_{12})$-Alkenylen- oder $(C_3-C_{12})$-Alkinylenkette und

Z CH oder N bedeuten.

2. Mittel gemäß Anspruch 1, gekennzeichnet durch einen Gehalt an einer Verbindung der Formel I, worin

Ar die Gruppe

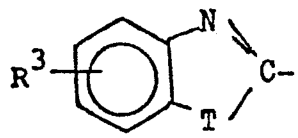 oder 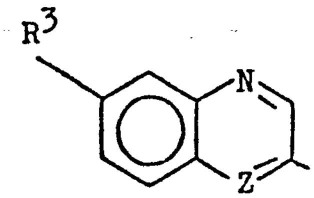

$R^1$ Wasserstoff oder $(C_1-C_6)$-Alkyl

$R^2$ Wasserstoff

$R^3$ Wasserstoff, Fluor, Chlor, Brom oder $CF_3$,

T O oder S,

X O

Z CH oder N,

n 1, 2, 3 oder 4 und

Y eine geradkettige oder verzweigte $(C_2-C_{12})$-Alkylenkette

bedeuten.

3. Mittel gemäß Anspruch 2, dadurch gekennzeichnet, daß n = 1 und $R_2$ Wasserstoff ist und Y für die Gruppe

$$-\underset{\displaystyle R^4}{\underset{|}{CH}}-(CH_2)_p-$$

steht,

in der $R^4$ Wasserstoff oder $CH_3$ und p 1 oder 2 bedeuten.

4. Verfahren zur Bekämpfung von unerwünschtem Pflanzenwuchs bzw. zur Wachstumsregulierung von Nutzpflanzen, dadurch gekennzeichnet, daß die Pflanzen oder die Anbauflächen mit einer wirksamen Menge einer Verbindung der Formel I behandelt werden.

5. Verfahren zur Herstellung der Wirkstoffe gemäß Anspruch 1, dadurch gekennzeichnet, daß man

a) eine Verbindung der Formel II

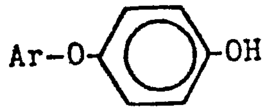

(II)

mit einer Verbindung der Formel III,

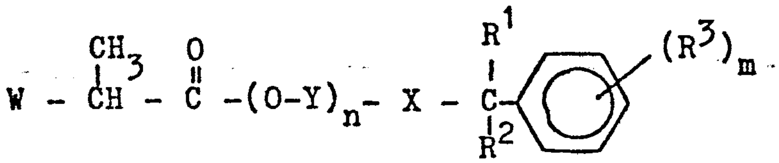

(III)

worin W für ein Halogenatom, eine Mesyl- oder Tosylgruppe steht, oder

b) eine Verbindung der Formel IV

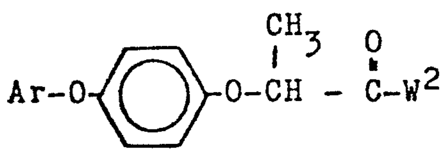

(IV)

worin $W^2$ für eine Hydroxylgruppe oder Halogenatom steht, mit einer Verbindung der Formel V

mit einer Verbindung der Formel V

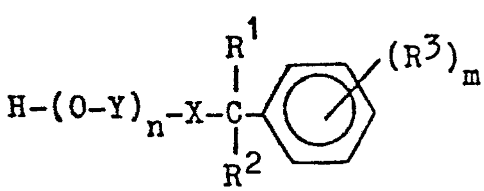

(V)

oder

c) eine Verbindung der Formel VI

Ar-W (VI)

mit einer Verbindung der Formel VII

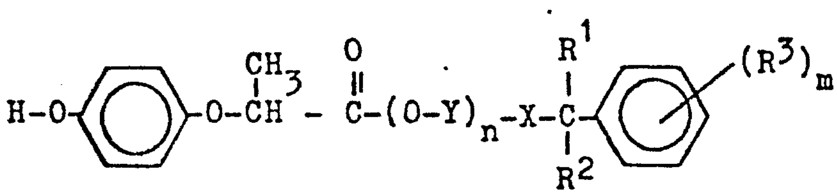

(VII)

umsetzt.